# EUROPEAN PATENT APPLICATION

(11) **EP 4 166 658 A1**
(43) Date of publication of application: **19.04.2023**
(21) Application number: 22201364.1
(22) Date of filing: 13.10.2022
(51) Int. Cl.: C12N 9/02, C12P 13/24

(54) **DIATOM-BASED GENETIC ENGINEERING SYSTEM METHODOLOGY FOR THE ECO-SUSTAINABLE PRODUCTION OF OVOTHIOLS**

(30) Priority: 18.10.2021 IT 202100026639
(71) Applicant: Stazione Zoologica Anton Dohrn, 80121 Napoli (NA) (IT)
(72) Inventor: Castellano, Immacolata, 80121 Napoli (IT); Russo, Monia, Teresa, 80121 Napoli (IT); Ferrante, Maria, Immacolata, 80121 Napoli (IT); Palumbo, Anna, 80121 Napoli (IT)
(74) Representative: Di Giovine, Paolo

(57) **Abstract**

The present invention relates to the field of biotechnology, in particular it relates to the set-up of a protocol of enzymatic engineering of the diatom species *Phaeodactylum tricornutum* to overexpress the biosynthetic enzyme leading to ovothiol production. This protocol is eco-sustainable because it uses cells and nutrients for biosynthesis and does not produce toxic side-compounds. Microalgal biomass moreover can be exploited for additional uses after ovothiol extraction, making the production process more cost-effective. The production of ovothiols is relevant for the pharmaceutical, nutraceutical, and cosmeceutical sectors.

## Description

### Technical field

The present invention relates to the field of biotechnology, in particular it relates to the set-up of a protocol of enzymatic engineering of the diatom species *Phaeodactylum tricornutum* to overexpress the biosynthetic enzyme leading to ovothiol production. This protocol is eco-sustainable because uses cells and nutrients for biosynthesis and does not produce toxic side-compounds. Microalgal biomass moreover can be exploited for additional uses after ovothiol extraction, making the production process more cost-effective. The production of ovothiols is relevant for the pharmaceutical, nutraceutical, and cosmeceutical sectors.

### Background

The compounds of natural origin to be used for the design of new drugs in order to overcome the side effects or the lack of efficacy of chemical synthesis products are of considerable interest in the chemical and pharmaceutical sector.

Ovothiols (5-thiohistidine derivatives) of marine origin have recently attracted the research community's interest for their potential to treat a plethora of pathologies, for example to prevent and treat low-grade chronic systemic inflammation (ISC) and ISC-related diseases which include: chronic inflammatory diseases, autoimmune diseases, chronic degenerative diseases, ISC-related tumors, obesity, diabetes, arterial hypertension, hypercholesterolemia, cardio-vascular diseases, osteoporosis, respiratory diseases and polycystic ovary syndrome (Patent PCT N. PCT/IB2018/057098). Ovothiols have also been proposed for the prevention and treatment of gamma-glutamyl-transpeptidase (GGT)-related diseases in which the GGT-related pathologies are selected from the group consisting of: renal ischemia/reperfusion damage, diseases associated with reverse airway obstruction, pathologies related to the increase in GGT activity levels in the blood (Patent EP19210282). Ovothiol A has also been shown to have anti-proliferative activity in human hepatocarcinoma and leukemia cell lines. (Brancaccio, M., Russo, M., Masullo, M., Palumbo, A., Russo, G.L., Castellano, I., 2019, Sulfur-containing histidine compounds inhibit γ-glutamyl transpeptidase activity in human cancer cells. J. Biol. Chem., 294, 14603-14614).

Thiohistidines are a class of sulfur-containing amino acids with key properties in the removal of peroxides (Holler, T.P. and Hopkins, P.B., 1988, Ovothiols as biological antioxidants. The thiol groups of ovothiol and glutathione are chemically distinct. J. Am. Chem. Soc., 110, 4837-4838; Bailly, F., Vezin, H., Teissier, E., Duriez, P., Fruchart, J.C., Catteau, J.P., Bernier, J.L., 2000, 4-Mercaptoimidazoles derived from the naturally occurring antioxidant ovothiols 1. Antioxidant properties. Free Radic. Res., 32, 515-524).

Among these, 2-thiohistidines called ergothioneine are present mainly in some fungi and bacteria whereas 5-thiohistidines are present mainly in marine invertebrates, bacteria and microalgae. These natural sulfur-containing products occur both as free amino acids and as constituents of an iron chelating pigment or an alkaloid (reviewed in Palumbo, A., Castellano, I., Napolitano, A., 2018, Ovothiol: a potent natural antioxidant from marine organisms. In Blue Biotechnology. Production and use of marine molecules; Castellano, I, and Seebeck, F,P., 2018, On ovothiol biosynthesis and biological roles: from life in the ocean to therapeutic potential. Nat. Prod. Rep. 35, 1241-1250). The 5-thiohistidines, thanks to the peculiar position of the thiol group on the imidazole ring of histidine, are probably the most acidic thiols among natural products and are also endowed with unique redox properties. Ovothiols, π-methylated 5-thiohistidines, can play a key role in controlling cellular redox balance, thanks to their ability to carry out redox exchange with glutathione. Ovothiol A (1) is a π-methyl-5-thiohistidine, isolated and characterized from the eggs of the sea urchin *Paracentrotus lividus* and other marine invertebrates. Ovothiol A has also been recently identified in the green microalgae *Euglena gracilis,* which is known for its potential in biotechnological applications, thanks to the easy cultivation and the high diversity of the metabolic profile. Ovothiol B (2), methylated also at the lateral side chain has been recently discovered in the diatom *Skeletonema marinoi* (Milito, A., Castellano, I., Burn, R., Seebeck, F.P., Brunet, C., Palumbo, A., 2020, First evidence of ovothiol biosynthesis in marine diatoms. Free Radic. Biol. Med. 152, 680-688). Ovothiol C (3), di-methylated at the amino acidic amino group, has been isolated from other sea urchin species.

Ovothiols are biosynthesized by three enzymatic reactions. The enzymatic production begins with oxidative carbon-sulfur (C-S) bond formation between cysteine and the C5 position on the histidine imidazole (EC 1.14.99.52). Subsequent removal of the cysteinyl moiety and methylation of the imidazole ring lead to the final product ovothiol. The key enzyme involved in ovothiol biosynthesis is a bifunctional enzyme, which catalyzes the formation of the oxidative C-S bond, which results in the net sulfur transfer from cysteine to position 5 of histidine. In detail, the enzyme 5-histidylcysteine sulfoxide synthase (OvoA) catalyzes the formation of the 5-histidyl-cysteine sulfoxide conjugate from cysteine and histidine (Braunshausen, A. and Seebeck, F.P., 2011, Identification and Characterization of the First Ovothiol Biosynthetic Enzyme. J. Am. Chem. Soc. 133, 1757-1759). Subsequently, a pyridoxal phosphate (PLP)-dependent lyase (OvoB) cleaves this intermediate product to generate 5-thiohistidine. Then, OvoA catalyzes the methylation at the imidazole ring to produce ovothiol A.

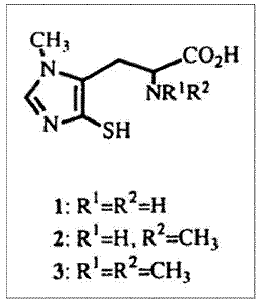

However, currently no company produces and sells ovothiols because the protocols of chemical synthesis described so far are too cumbersome and expensive for a convenient commercial production of these molecules.

On the other hand, the precursor of ovothiols, non-methylated 5-thiohistidine, can be prepared by a more feasible chemical synthesis according to the protocol described by Daunay et al. (2016) "Short protecting-group-free synthesis of 5-acetylsulfanyl-histidines in water: novel precursors of 5-sulfanyl-histidine and its analogues". Org. Biomol. Chem. 14, 10473-10480.

The π-methylated derivatives, ovothiols, instead can be purified by sea urchin eggs. In particular, ovothiol A can be extracted and purified from the sea urchin *Paracentrotus lividus* eggs according to the protocol described by Russo et al. (2014) "Ovothiol isolated from sea urchin oocytes induces autophagy in the Hep-G2 cell line." Mar. Drugs, 12, 4069-4085. The amount of 2.5 mg of pure ovothiol A can be obtained from 10 g of fresh eggs. However, sea urchins are not an eco-sustainable source for these compounds and cannot provide sufficient amounts of ovothiols for extensive studies, also considering the necessity to preserve natural populations at sea.

The U.S. Pat. US 4898878 describes active ovothiol derivatives in which the substituents are individually selected from hydrogen, methyl or other atoms and groups that do not negatively influence the overall redox activity spectrum of the molecule. Moreover, the chemical synthesis of ovothiols is also reported involving numerous steps (10-12) leading to the initial formation of the 4-thioimidazole ring precursor and subsequent generation of the amino acid side chain. However, the procedure is time- and cost-consuming.

The U.S. PATENT 9,926,300B2 describes the chemical synthesis of 5-acylsulfanyl-histidine compounds through direct introduction of an acylsulfanyl group in position 5 of histidine or of one of its derivatives. These compounds are capable of being precursors of the corresponding 5-sulfanylhistidines and their disulfides. By this procedure, the synthesis of ovothiols which are characterized by the methyl group at position π of the imidazole ring is not feasible. By contrast, iso-ovothiols, which are methylated at position τ of the imidazole ring, are synthesized.

The U.S. Patent 2020/0017479 A1 is in part a continuation of the U.S. PATENT 9,926,300B2. It describes the synthesis of 5-acylsulfanyl-histidine compounds and their use as precursors of the corresponding 5-sulfanylhistidines and their disulfides. Also, the antioxidant activity of 5-sulfanylhistidines derivatives is tested measuring GSH-Px activity. By this procedure, ovothiols are not synthesized. Only iso-ovothiols which are methylated at position τ of the imidazole ring are synthesized.

The U.S. Patent Application No. 20100168198 describes a pro-antioxidant drug targeted to mitochondria, useful for the prevention or treatment of diseases or conditions associated with mitochondrial dysfunction resulting from changes in the mitochondrial redox environment. The pro-drugs are produced by modifying an antioxidant in a fatty acid so that the resulting pro-drug is targeted and activated by an enzyme responsible for the beta-oxidation of mitochondrial fatty acids. The examples describe the activation of beta-oxidation of fatty acids of the anti-oxidant pro-drugs based on 4-mercaptoimidazole (ovothiol).

An alternative natural source for ovothiol biosynthesis can be microalgae, and especially diatoms.

The microalga *Phaeodactylum tricomutum,* one of the most studied models microalgal species, with a fully sequenced genome and a number of tools for genetic engineering, belongs to the group of diatoms, photosynthetic unicellular eukaryotes with a complex evolutionary origin that confers them features of plants as well as animals. Due to the ease of cultivation and the high biomass yield, *P. tricornutum* is a promising system in industrial applications where it can be grown for several applications (Butler, T., Kapoore, R.V., Vaidyanathan, S., 2020, A Diatom Cell Factory. Trends Biotechnol. 38, 606-622). During the last two decades, significant efforts have been devoted to the development of molecular tools to study diatom biology, as well as to establish these algae as a significant, renewable and sustainable resource of biomass for feed, food, energy, and other value-added products. The development of tools for genetic manipulation of diatoms is paving the way for a growing exploitation of these organisms in biotechnology, as feedstock for biofuels and applications for food, human health or green chemistry.

Diatoms can be genetically transformed to produce high amounts of an endogenous product or can be used as a cell factory aimed at the production of exogenous molecules of interest. Exogenous genes can be inserted in *P. tricornutum* with different methods: the most common method is the biolistic bombardment of plasmid DNA which enters the cells on metal particles and becomes integrated into the host genome; electroporation has found a limited use and it has never proven to be efficient, while a more recent method based on bacterial conjugation is becoming widespread (Karas, B., Diner, R., Lefebvre, S. et al. 2015 Designer diatom episomes delivered by bacterial conjugation. Nat Commun 6, 6925; Diner, R.E., Bielinski, V.A., Dupont, C.L., Allen, A.E., Weyman, P.D., Refinement of the Diatom Episome Maintenance Sequence and Improvement of Conjugation-Based DNA Delivery Methods. Front. Bioeng. Biotechnol. 4, 65). With this latter method, the capability of bacteria to conjugate with diatoms is exploited: the exogenous DNA is cloned in a specific vector and is introduced in an *Escherichia coli* strain that is capable to transfer it to the diatom via conjugation. The vector has specific sequences that allow it to be maintained in the diatom as an episome, an extrachromosomal circular DNA which does not integrate in the endogenous genome but can be duplicated and inherited by daughter cells.

Several patents exist for *P. tricornutum* model species, mostly with the aim of increasing the growth rate for productivity purposes by modification of media or light incubation properties. Standing out for the enrichment of very-long-chain polyunsaturated fatty acids and high contents of neutral lipids, this species is highly exploited for the modification of enzymes involved in lipid metabolism to increase lipid content. Functional to gene expression modulation, there are several patents regarding the isolation of strong constitutive or inducible promoters that are conveniently used for the creation of overexpression constructs.

However, up to date, no protocols have been described for a sufficient synthesis of ovothiol compounds by microalgae.

### Summary of the invention

The present invention concerns the set-up of a protocol of enzymatic engineering of the diatom species *Phaeodactylum tricornutum* to overexpress the biosynthetic enzyme leading to ovothiol production. The authors of the present invention have verified that the cells of the modified strains produce higher amounts of ovothiol B compared to wild type. The use of *P. tricornutum* cultures has many advantages over other non-conventional sources; they accomplish high productivity and allow easy scale-up of upstream and downstream processes and are little influenced by seasonal changes. Biomass can be easily obtained in the laboratory and *P. tricornutum* cultures can be grown in bioreactors for the production of high valuable compounds.

This protocol is eco-sustainable because it uses cells and nutrients for biosynthesis and does not produce toxic side-compounds. Microalgal biomass moreover can be exploited for additional uses after ovothiol extraction, making the production process more cost-effective. The production of ovothiols is relevant for the pharmaceutical, nutraceutical, and cosmeceutical sectors.

The system discovered by the Authors of the invention is competitive because it allows to produce ovothiols (π-methylated derivatives) in an eco-sustainable way without production of toxic compounds and expensive procedures. Indeed, while a chemical synthesis for the production of the un-methylated form 5-thiohistidine is described and feasible (Daunay et al. 2016), the old described procedure for the synthesis of the π-methylated forms (ovothiols) is time-consuming and not-environmental friendly (Holler, T.P., Ruan, F., Spaltenstein, A., and Hopkins, P.B., 1989, Total synthesis of marine mercaptohistidines: ovothiols A, B, and C. J. Org. Chem. 54, 4570-4575). Moreover, ovothiol A can be extracted and purified from the sea urchin *P. lividus* eggs according to the protocol described by Russo et al. 2014. However, sea urchins cannot provide sufficient amounts of ovothiols for extensive studies (2.5 mg of pure ovothiol A obtained from 10 g of fresh eggs), also considering the necessity to preserve natural populations of marine invertebrate species. Actually, no company produce and sells ovothiols.

Therefore, objects of the present invention are:
a genetically modified microalgae, in particular *P. tricornutum,* characterized in that said microalgae includes one or more copy of an overexpression cassette encoding for the 5-histidylcysteine sulfoxide synthase OvoA enzyme, wherein said 5-histidylcysteine sulfoxide synthase OvoA enzyme has SEQ ID NO: 17 or isoforms or mutant sequences thereof with a sequence identity between 90-99% and the same enzymatic activity;
a method for the preparation of a genetically modified microalgae, in particular P. *tricornutum,* comprising a step of transformation of said microalgae with an exogenous sequence encoding for the 5-histidylcysteine sulfoxide synthase OvoA enzyme, wherein said 5-histidylcysteine sulfoxide synthase OvoA enzyme has SEQ ID NO: 17 or isoforms or mutant sequences thereof with a sequence identity between 90-99% and the same enzymatic activity;
an expression vector suitable for the transformation of microalgae, in particular P. *tricornutum,* comprising a nucleic acid sequence encoding for the 5-histidylcysteine sulfoxide synthase OvoA enzyme, wherein said nucleic acid sequence encoding for the sulfoxide synthase OvoA enzyme has SEQ ID NO: 17 or isoforms or mutant sequences thereof with a sequence identity between 90-99% and the same enzymatic activity, and a method for the identification and/or quantification of ovothiol(s) comprising the steps of:
   i) culturing a genetically modified microalgae, in particular *P. tricornutum,*
   ii) harvesting said genetically modified microalgae and
   iii) identification and analysis of the ovothiol(s) from said genetically modified microalgae obtained on step ii).

### Brief description of the drawings

The present invention and the following detailed description of preferred embodiments thereof may be better understood with reference to the following figures:

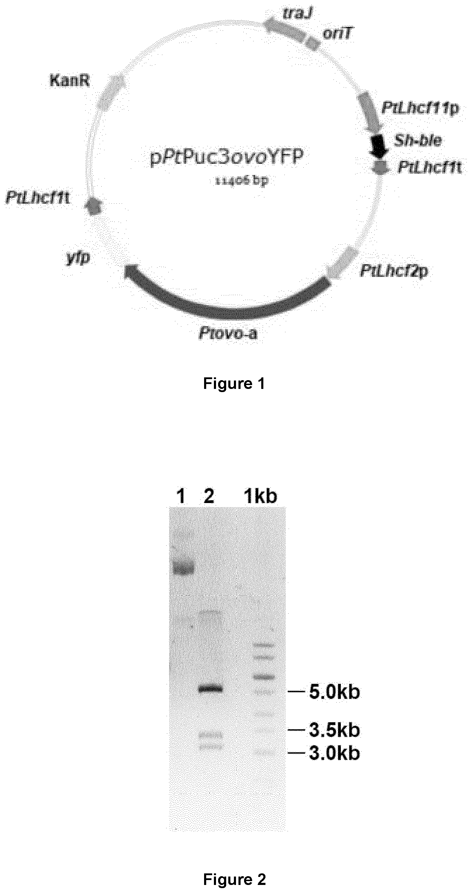

**Figure 1****. Expression construct for bacterial conjugation.** The cargo vector (pPtPuc3ovoYFP) included an expression cassette for antibiotic (Sh-ble) selection, a cassette for the overexpression of *P. tricornutum* PtOvoA fused to YFP. *PtLhcf11p, P*. *tricornutum Lhcf11* promoter; *PtLhcf1t, P. tricornutum Lhcf1* terminator; *PtLhcf2p, P. tricornutum Lhcf2* promoter; PtOvoA, P. *tricornutum* OvoA coding sequence; KanR, resistance to Kanamycin; oriT, origin of transfer; traJ, oriT recognzing protein.
**Figure 2****. Digestion of the bacterial conjugation construct.** Electrophoretic analysis of the restriction digestion of an *E. coli* resistant clone plasmid showing bands of 5026, 3316 and 3064 bp. lane 1, undigested plasmid; lane 2, Clal digested plasmid; 1kb, molecular weight ladder.

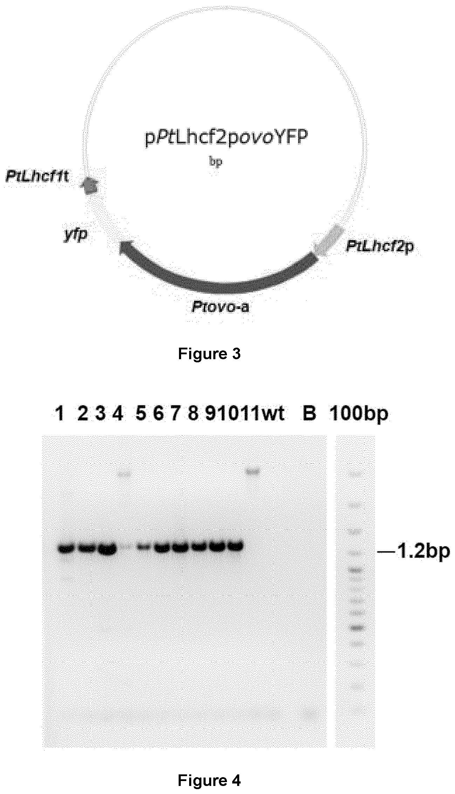

**Figure 3****. Expression construct for biolistic transformation.** The pPtLhcf2povoYFP construct included a cassette for the overexpression of *P*. *tricornutum* PtOvoA fused to YFP. PtLhcf2p, *P*. *tricornutum* Lhcf2 promoter; PtLhcf1t, *P*. *tricornutum* Lhcf1 terminator; PtOvoA, *P*. *tricornutum* OvoA coding sequence.
**Figure 4** **Colony PCR of clones obtained by bacterial conjugation.** Electrophoresis of resistant clones (lanes 1-11), positive clones showed a band of 1205 bp. wt, wild type; B, blank (no template); 100bp, molecular weight ladder.

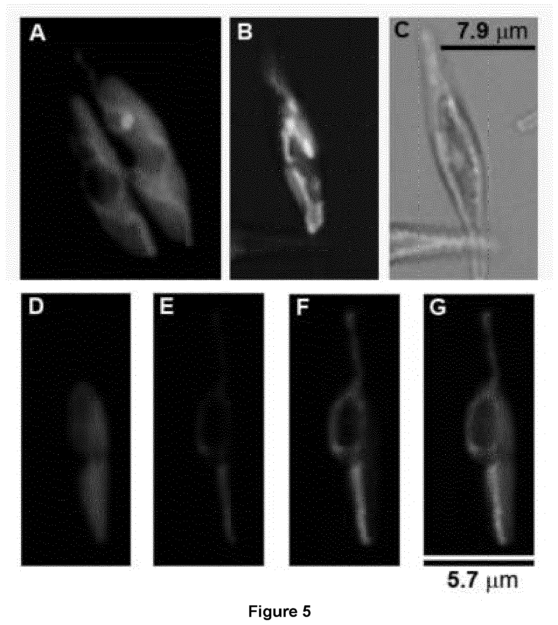

**Figure 5****. Cellular localization of the clones obtained by bacterial conjugation.** *P*. *tricornutum* OvoA-overexpressing cells showing a signal corresponding to the autofluorescing chlorophyll and to the fusion protein PtOvoA-YFP (A-B), bright field of the cell in B (C). In the bottom panel the signals are separated in the autofluorescing chlorophyll (D), the mitochondrion staining marker (E) and the fusion protein PtOvoA-YFP (F). The different signals are merged in G.

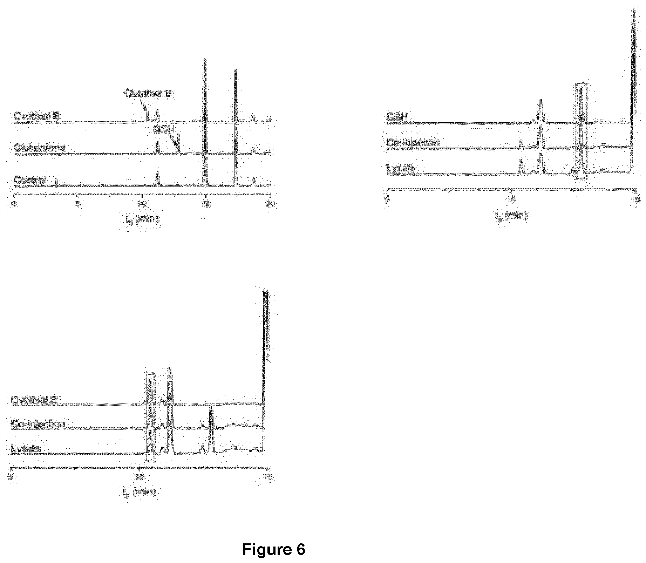

**Figure 6****. HPLC analysis of cellular thiols in *P. tricornutum* cells.** Top left: HPLC traces of authentic samples of ovothiol B and glutathione. Top right: co-injection of authentic glutathione sample with the lysate. The box highlights the peaks corresponding to glutathione. Bottom left: co-injection of the authentic ovothiol B sample with the lysate. The box highlights the peaks corresponding to ovothiol B.

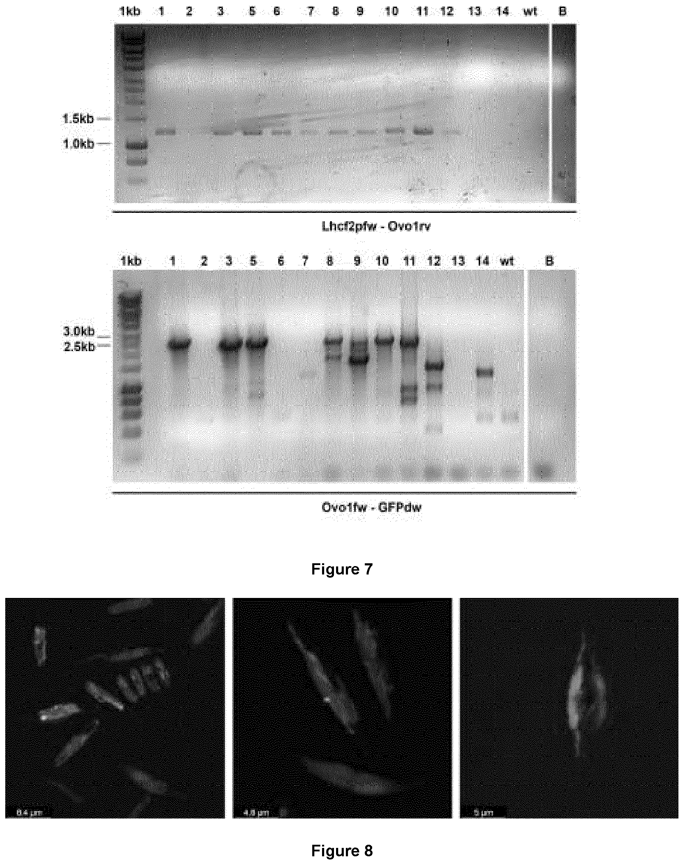

**Figure 7****. Colony PCR of clones obtained by biolistic transformation.** Electrophoresis of resistant clones (lanes 1-14) Positive clones showed the expected bands of 1237 bp (top panel, lanes 1, 3-12) and 2619 bp (bottom panel, lanes 1, 3, 5, 8-11). Additional smaller bands in the bottom panel indicate the possible presence of truncated versions of the construct. wt, wild type; B, blank (no template); 1kb, molecular weight ladder.
**Figure 8****. Cellular localization of the clones obtained by biolistic transformation.** Confocal laser scanning microscopy images of *P. tricornutum* OvoA-overexpressing cells showing a signal corresponding to the autofluorescing chlorophyll (diffused gray) and a signal corresponding to the fusion protein PtOvoA-YFP (bright spots and bright gray threads).

### Detailed description

In the following, several embodiments of the invention will be described. It is intended that the features of the various embodiments can be combined, where compatible. In general, subsequent embodiments will be disclosed only with respect to the differences with the previously described ones.

First object of the present invention is a genetically modified microalga, characterized in that said microalga includes one or more copy of an expression cassette encoding for the 5-histidylcysteine sulfoxide synthase OvoA enzyme, wherein said nucleic acid sequence encoding for the sulfoxide synthase OvoA enzyme has SEQ ID NO: 17 or isoforms or mutant sequences thereof with a sequence identity between 90-99% and the same enzymatic activity.

According to the invention, "genetically modified" means any modification of the genome of the microalgae obtained by human intervention or under human control consisting in introducing a heterologous/exogenous nucleic acid sequence into the algae, either into the genome or maintained as extrachromosomal circular DNA in the cytoplasm of the microalgae. The result of this modification is to enrich the genome of the genetically modified microalga with the addition of new sequences.

The terms "polynucleotide" and "nucleic acid" are used interchangeably herein and generally refer to a polymer of any length composed essentially of nucleotides, e.g., deoxyribonucleotides and/or ribonucleotides. Nucleic acids can comprise purine and/or pyrimidine bases, and/or other natural, chemically or biochemically modified (e.g., methylated), non-natural, or derivatized nucleotide bases. The backbone of nucleic acids can comprise sugars and phosphate groups, as can typically be found in RNA or DNA, and/or one or more modified or substituted (such as, 2'-0-alkylated, e.g., 2 -0- methylated or 2'-0-ethylated; or 2'-0,4'-C-alkylated, e.g., 2'-0,4'-C-ethylated) sugars or one or more modified or substituted phosphate groups. For example, backbone analogues in nucleic acids may include phosphodiester, phosphorothioate, phosphorodithioate, methylphosphonate, phosphoramidate, alkyl phosphotriester, sulfamate, 3'-thioacetal, methylene (methylimino), 3'-N-carbamate, morpholino carbamate, and peptide nucleic acids (PNAs). The term nucleic acid further specifically encompasses DNA, RNA and DNA RNA hybrid molecules, specifically including hnRNA, pre-mRNA, mRNA, cDNA, genomic DNA, gene, amplification products, oligonucleotides, and synthetic (e.g. chemically synthesized) DNA, RNA or DNA RNA hybrids. The terms "ribonucleic acid" and "RNA" as used herein mean a polymer of any length composed of ribonucleotides. The terms "deoxyribonucleic acid" and "DNA" as used herein mean a polymer of any length composed of deoxyribonucleotides. The term "DNA/RNA hybrid" as used herein mean a polymer of any length composed of one or more deoxyribonucleotides and one or more ribonucleotides. A nucleic acid can be naturally occurring, e.g., present in or isolated from nature, can be recombinant, i.e., produced by recombinant DNA technology, and/or can be, partly or entirely, chemically or biochemically synthesized. A nucleic acid can be double-stranded, partly double stranded, or single-stranded. Where single-stranded, the nucleic acid can be the sense strand or the antisense strand. In addition, nucleic acid can be circular or linear.

As used herein, the term "nucleic acid expression cassette" refers to nucleic acid molecules that include one or more transcriptional control elements (such as, but not limited to promoters, enhancers, polyadenylation sequences, and introns) that direct expression of a (trans)gene(s) to which they are operably linked.

The term "operably linked" as used herein refers to the arrangement of various nucleic acid molecule elements relative to each such that the elements are functionally connected and are able to interact with each other in the context of gene expression. Such elements may include, without limitation, a promoter, an enhancer, a polyadenylation sequence, one or more introns, and a coding sequence of a gene of interest to be expressed (e.g., the (trans)gene). The nucleic acid sequence elements, when properly oriented or operably linked, act together to ensure or modulate expression of the coding sequence. By modulate is meant increasing, decreasing, or maintaining the level of activity of a particular element. The position of each element relative to other elements may be expressed in terms of the 5' terminus and the 3' terminus of each element, and the distance between any particular elements may be referenced by the number of intervening nucleotides, or base pairs, between the elements.

The term "microalgae" as used herein refers to microscopic algae. "Microalgae" encompass, without limitation, organisms within (i) several eukaryotic phyla, including the *Rhodophyta* (red algae), *Chlorophyta* (green algae), *Dinoflagellata, Haptophyta,* (ii) several classes from the eukaryotic phylum *Heterokontophyta* which includes, without limitation, the classes *Bacillariophycea* (diatoms), *Eustigmatophycea, Phaeophyceae* (brown algae), *Xanthophyceae* (yellow-green algae) and *Chrysophyceae* (golden algae), and (iii) the prokaryotic phylum Cyanobacteria (blue-green algae). The term "microalgae" includes for example genera selected from: *Achnanthes, Amphora, Anabaena, Anikstrodesmis, Arachnoidiscusm, Aster, Botryococcus, Chaetoceros, Chlamydomonas, Chlorella, Chlorococcum, Chorethron, Cocconeis, Coscinodiscus, Crypthecodinium, Cyclotella, Cylindrotheca, Desmodesmus, Dunaliella, Emiliana, Euglena, Fistulifera, Fragilariopsis, Gyrosigma, Hematococcus, Isochrysis, Lampriscus, Monochrysis, Monoraphidium, Nannochloris, Nannochloropsis, Navicula, Neochloris, Nephrochloris, Nephroselmis, Nitzschia, Nodularia, Nostoc, Odontella, Oochromonas, Oocystis, Oscillartoria, Pavlova, Phaeodactylum, Playtmonas, Pleurochrysis, Porhyra, Pseudoanabaena, Pyramimonas, Scenedesmus, Schyzochitrium, Stichococcus, Synechococcus, Synechocystis, Tetraselmis, Thalassiosira, and Trichodesmium.* 5-histidylcysteine Sulfoxide synthase OvoA (EC 1.14.99.52) is an enzyme that participates in ovothiol biosynthesis, catalyzing the chemical reaction below

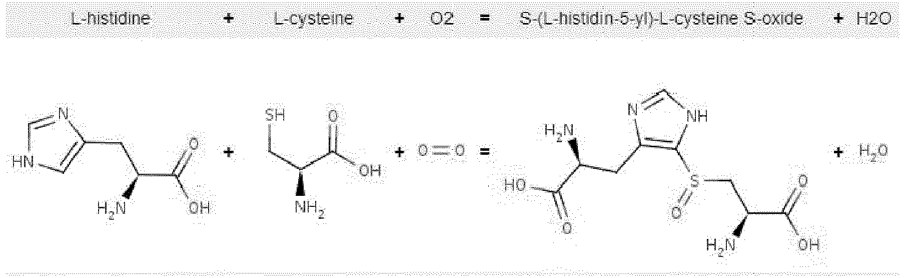

The term "exogenous" refers to introduction of a referenced molecule (e.g., nucleic acid such as DNA or mRNA, but also the protein) or referenced activity into a host cell. In particular, a nucleic acid may exogenously be introduced into a host in any suitable manner. For example, a nucleic acid can be introduced into a host cell and inserted into a host chromosome, or the nucleic acid can be introduced into the host as non-chromosomal genetic material, such as a vector (e.g., a plasmid) that does not integrate into the host chromosome. A nucleic acid encoding a protein may be introduced in an expressible form (i.e., so that the nucleic acid can be transcribed and translated). An exogenous "activity" (e.g., biosynthesis activity) refers to an activity introduced into a host cell, such as by introducing one or more nucleic acids to the host that are expressed to provide the activity.

In one embodiment, said microalga is a diatom.

In a preferred embodiment of the invention, said diatom is of the species *Phaeodactylum tricomutum.*

In a particularly preferred embodiment, said diatom is the strain Axenic CCMP632 of *P. tricornutum.*

In an embodiment of the present invention, said microalga exhibits improved expression of the sulfoxide synthase OvoA enzyme as compared to the wild type of said microalga.

The expression wild-type (WT) refers to the phenotype of the typical form of a species as it occurs in nature.

The term "improved expression" refers to a detectable increase in expression of a protein or an enzyme. The term "improved expression" used herein may mean that a modified (for example, genetically engineered) protein or enzyme shows higher expression than a comparable protein or enzyme of the same type, such as a protein or an enzyme which does not have a particular genetic modification (e.g., an original or wild-type protein or enzyme, or the expression level of a protein or enzyme of a host cell that served as the starting point for genetic modification). For example, expression of a modified or engineered protein or enzyme may be higher than expression of a non-engineered protein or enzyme of the same type (e.g. a wild-type protein or an enzyme, or the expression exhibited by a protein or enzyme of a host cell) by about 5% or more, about 10% or more, about 15% or more, about 20% or more, about 30% or more, about 50% or more, about 60% or more, about 70% or more, or about 100% or more. A host cell having a protein or enzyme having an improved enzymatic expression may be verified by any methods known in the art.

In a further embodiment of the invention, said microalga exhibits improved production of ovothiols as compared to the wild type of said microalga.

The expression "improved production" refers to a detectable increase in production of a molecule. The term "improved production" used herein may mean that a modified (for example, genetically engineered) organism shows higher production than a comparable organism of the same type, such as a microalga which does not have a particular genetic modification (e.g., an original or wild-type microalga). In one embodiment such improved production is at least two folds compared to the wild type by using the method of conjugation, and it ranges from three to five folds in clones produced by using the biolistic method.

In another embodiment, according to the present invention, said ovothiol is Ovothiol A, Ovothiol B or Ovothiol C, preferably Ovothiol B.

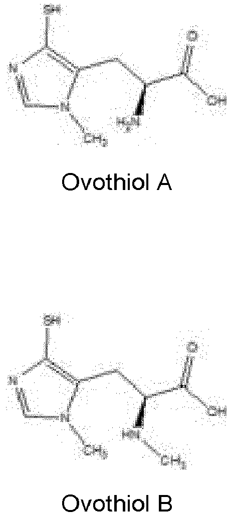

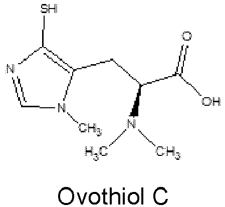

Sequence homology is the biological homology between DNA, RNA, or protein sequences, defined in terms of shared ancestry in the evolutionary history of life. Two segments of DNA can have shared ancestry because of three phenomena: either a speciation event (orthologs), or a duplication event (paralogs), or else a horizontal (or lateral) gene transfer event (xenologs). Homology among DNA, RNA, or proteins is typically inferred from their nucleotide or amino acid sequence similarity. Significant similarity is strong evidence that two sequences are related by evolutionary changes from a common ancestral sequence. Alignments of multiple sequences are used to indicate which regions of each sequence are homologous. The skilled person will be able to identify other sequences homologous to SEQ ID NO 17 from known sequences or those obtained from the genome of microalgae and by conventional single or multiple alignment methods such as the Clustal methods (Sievers, F., Wilm, A., Dineen, D., et al., 2011, Fast, scalable generation of high-quality protein multiple sequence alignments using Clustal Omega. Mol. Syst. Biol. 7, 539) or based on the Smith-Waterman method with Blossum 62 comparison matrix (Rice et al., 2000). Homologous sequences suitable for the invention are sequences that have the same enzymatic activity. Preferably the term "substantially homologous" refers to two sequences which are at least 90%, 95%, 99% identical in sequence, as measured by the BestFit program described herein (Version 10; Genetics Computer Group, Inc., Madison, Wis.), using default parameters.

In another embodiment, wherein wherein said overexpression cassette encoding for 5-histidylcysteine sulfoxide synthase OvoA enzyme comprises a nucletodic sequence having SEQ ID NO: 18..

The sequence encoding for the 5-histidylcysteine sulfoxide synthase OvoA has been filed in GenBank with the identification number OP503499,

In one embodiment of the present invention, said genetically modified microalga is obtainable by the any of method herein described below.

As previously anticipated, further object of the present invention is a method of the preparation of a genetically modified microalga, comprising a step of transformation of said microalga with an exogenous sequence encoding for the 5-histidylcysteine sulfoxide synthase OvoA enzyme, wherein said sulfoxide synthase OvoA enzyme has SEQ ID NO: 17 or isoforms or mutant sequences thereof with a sequence identity between 90-99% and the same enzymatic activity.

The word "transformation" is referred to the process of altering a cell's genetic code through the uptake of foreign DNA from the environment. Genetic transformation is the process by which exogenous DNA is transferred into the host cell. Transformation usually implies uptake of DNA into bacterial, yeast or plant cells.

In an embodiment of the invention, the method comprises a step of transformation of said microalgae with an expression vector comprising nucleotide sequence encoding for the 5-histidylcysteine sulfoxide synthase OvoA enzyme.

The word "vector" refers to any vehicle, often a virus or a plasmid, that is used to ferry a desired DNA sequence into a host cell as part of a molecular cloning procedure. Depending on the purpose of the cloning procedure, the vector may assist in multiplying, isolating, or expressing the foreign DNA insert.

In an embodiment, the expression vector can be selected among the following ones: a plasmid, a yeast vector, a mammalian vector, a viral vector, a single-stranded phage, a double-stranded phage, an artificial chromosome and/or a combination thereof.

In a preferred embodiment, in accordance with any one of the embodiments herein disclosed, the expression vector is a plasmid, in particular pPtPuc3 and pPtLhcf2pLAHYFP plasmids.

In a preferred embodiment of the invention, said expression vector is introduced in said microalgae by biolistic method or bacterial conjugation.

The biolistic method, also known as the "gene gun", involves firing small metal particles coated with the construct DNA into plant cells. The metal particles, usually gold or tungsten, are accelerated to high speed by the rapid release of high-pressure helium in the gene gun into the target cells. The target is totipotent cells, usually consisting of an embryogenic suspension culture or an embryogenic callus derived from the recipient plant. Some of the metal particles enter the nuclei of cells without causing lethal damage and deliver the construct so that it can integrate by recombination with the chromosomes. The target cells are then induced to form plants under selection which only permits material expressing the selectable marker (and usually the GOI) to survive. The main advantage of the biolistic method of gene transformation is that it is genotype independent because it uses physical force to insert DNA into plant tissues. One more advantage for biolistic delivery is that the gene of interest does not need to be cloned into a specialized transformation vector in order to be integrated into plant cells and subsequently inherited.

The solution proposed by the authors of the present invention, in order to produce an increased amount of ovothiol from *P. tricornutum* axenic cultures is the exploitation of the biolistic method.

Therefore, in an embodiment of the present invention, according to any of the embodiments herein disclose, said expression vector is introduced in said microalgae by biolistic method. This approach induces a random integration of a variable number of transgenes in the genome of *P. tricornutum.* The strongest expressing clone(s), containing a high number of transgenes in the genome, can be easily selected.

On the other hand, the transgene integration in the genome, occurring when the biolistic method is applied, becomes an advantage because the clones are stable and they do not need continuous antibiotic selection in the medium. Moreover, the opportunity to have multiple transgene insertions permits to isolate a highly strong expressing clone and hence to obtain a higher amount of final product.

In an embodiment of the invention, said biolistic method is performed using a Biolistic Particle Delivery System (Bio-Rad)

In an embodiment, said biolistic method is performed as described in (Falciatore, A., Casotti, R., Leblanc, C., Abrescia, C., Bowler, C., 1999, Transformation of Nonselectable Reporter Genes in Marine Diatoms. Mar Biotech 1, 239-251).

Bacterial conjugation is the transfer of genetic material between bacterial cells by direct cell-to-cell contact or by a bridge-like connection between two cells. This takes place through a pilus. It is a parasexual mode of reproduction in bacteria.

It is a mechanism of horizontal gene transfer as are transformation and transduction although these two other mechanisms do not involve cell-to-cell contact.

Classical *E. coli* bacterial conjugation is often regarded as the bacterial equivalent of sexual reproduction or mating since it involves the exchange of genetic material. However, it is not sexual reproduction, since no exchange of gamete occurs, and indeed no generation of a new organism: instead, an existing organism is transformed. During classical *E. coli* conjugation, the donor cell provides a conjugative or mobilizable genetic element that is most often a plasmid or transposon. Most conjugative plasmids have systems ensuring that the recipient cell does not already contain a similar element.

The genetic information transferred is often beneficial to the recipient. Benefits may include antibiotic resistance, xenobiotic tolerance or the ability to use new metabolites. Other elements can be detrimental and may be viewed as bacterial parasites.

In a preferred embodiment of the invention, said expression vector is an episome vector in said algal cell. Episomes, in eukaryotes, are extrachromosomal, closed circular DNA molecules of a plasmid or a viral genome origin, that are replicated autonomously in the host cell and therefore, they bear significant vector potential for the transfer of nucleic acids into cells. The use of episomes therefore is "cleaner" than other traditional methods since it avoids random integration in the genome and the consequent risks of disrupting endogenous genes.

The solution proposed by the authors of the present invention, in order to produce an increased amount of ovothiol from *P. tricornutum* axenic cultures is therefore the exploitation of bacterial conjugation to obtain the overexpression of the 5-histidylcysteine sulfoxide synthase OvoA from an episomal vector.

Therefore, in a particularly preferred embodiment according to the present description, said expression vector is introduced in said strain by bacterial conjugation.

In an embodiment, the bacteria cells used for the bacterial conjugation is selected from the following ones: *Escherichia coli* DH10B cells.

Furthermore, in an embodiment of the invention, the method comprises a step wherein said expression vector is transformed in said bacteria cells, preferably DH10B cells.

In another embodiment of the invention, the method further comprises a step wherein said bacteria cells, preferably DH10B cells, are inoculated one day before the conjugation for the overnight growth.

In a preferred embodiment, the bacterial conjugation between said bacteria cells, preferably DH10B cells, and *P. tricornutum* is performed as in Karas B., Diner R., Lefebvre S. et al. 2015 Designer diatom episomes delivered by bacterial conjugation. Nat Commun 6, 6925. Doi: 10.1038/ncomms7925.

By means of the method based on bacterial conjugation, the OvoA overexpressing cassette is cloned in a specific vector and is introduced in an *Escherichia coli* strain that is capable to transfer it to the diatom via conjugation. The vector has specific sequences that allow it to be maintained in the diatom as an episome, an extrachromosomal circular DNA which does not integrate in the endogenous genome but can be duplicated and inherited by daughter cells. The method therefore is "cleaner" than other traditional methods since it avoids random integration in the genome and the consequent risks of disrupting endogenous genes.

In another embodiment of the invention, said expression vector is co-transformed with a vector which expresses a gene conferring resistance to an antibiotic.

In one embodiment, said gene is the Sh Ble gene.

In a further embodiment of the invention, said antibiotic is selected from phleomycin.

In a preferred embodiment of the present invention, said expression vector is co-transformed with a vector which expresses the Sh Ble gene conferring resistance to the phleomycin antibiotic.

Forms part of the present invention, an expression vector suitable for the transformation of microalgae, comprising a nucleic acid sequence encoding for the 5-histidylcysteine sulfoxide synthase OvoA enzyme, wherein said nucleic acid sequence encoding for the sulfoxide synthase OvoA enzyme has SEQ ID NO: 18.

In an embodiment, said expression vector is selected from a plasmid, a yeast vector, a mammalian vector, a viral vector, a single-stranded phage, a double-stranded phage, artificial chromosome and/or a combination thereof.

In a preferred embodiment, said expression vector is a plasmid.

An embodiment of the present invention consists in said expression vector operatively linked to a gene encoding a fluorescent protein.

In an embodiment, said fluorescent protein is the yellow fluorescent protein (YFP) or the green fluorescent protein (GFP).

In another embodiment, wherein said fluorescent protein is the yellow fluorescent protein (YFP).

In a particularly preferred embodiment of the invention, the expression vector is an episomal expression vector.

In another embodiment of the invention, according to all the embodiments herein disclosed, said expression vector comprises an antibiotic expressing cassettes, and the coding sequence of a fluorescent protein, and the P. tricornutum cDNA for the expression of 5-histidylcysteine sulfoxide synthase OvoA enzyme without the stop codon.

In one embodiment, said at least one antibiotic expressing cassettes are selected from kanamycin and bleomycin.

In another embodiment of the invention, said expression vector comprises the pPtPuc3 plasmid comprising a kanamycin and bleomycin expressing cassettes, the expression cassette for the yellow fluorescent protein (YFP), and the *P. tricornutum* cDNA for the expression of the 5-histidylcysteine sulfoxide synthase OvoA enzyme without the stop codon. Finally, object of the present invention is represented by a method for the identification and/or quantification of ovothiol(s) comprising the steps of:
i) culturing a genetically modified microalgae according to the present invention,
ii) harvesting said genetically modified microalgae and
iii) identification and/or quantification of the ovothiol(s) from said genetically modified microalgae obtained on step ii).

Microalgae culture methods are well known to the skilled person. Particular mention may be made of the culture methods described in Guillard et al (1975). Of course, the skilled person will be able to adapt the culture conditions, notably the composition of the medium, the conditions for adding nutrients during the culture, the temperature and the lighting conditions, in order to promote biomass production.

Algae harvesting refers to the separation or detachment of algae from its growth medium. The harvesting method intensely depends on the physiology of the microalgae chosen, density and size of the microalgal cell, specifications of the final product and on allowability for reuse of the culture medium. Algae harvesting involves mechanical, chemical, biological and electrical based methods, for example coagulation/flocculation, flotation, electrical based processes, filtration and centrifugation. It is possible to combine two or more of these methods to obtain a greater separation rate at lower costs. Suitable purification of the ovothiol(s) from said genetically modified microalga can be carried out by methods known to the person skilled in the art such as by using lysis methods, extraction, ion exchange chromatography, vacuum rotary evaporation, lyophilization.

In an embodiment, said harvesting step ii) is performed for example by centrifugation.

In another embodiment, said method further comprises a step after step ii) and before step iii) wherein the harvested culture is frozen in liquid nitrogen.

In compliance with Art. 170bis of the Italian patent law it is herein declared that:
All experiments involving cells were carried out on commercially available cells with reference to the MGOM used in the described experiments, the obligations deriving from the national or EU regulations have been fulfilled, in particular according to d.I. of 12 april 2001 n.206 and 8 June 2003 n. 224.

In any part of the present description and claims, the term comprising can be substituted by the term "consisting of".

Examples are reported below which have the purpose of better illustrating the methodologies disclosed in the present description, such examples are in no way to be considered as a limitation of the previous description and the subsequent claims.

### Examples

### METHODS

### Preparation of the overexpressing construct for bacterial conjugation

To prepare the pPtPuc3ovoYFP **(****Fig. 1****)** the Gibson assembly methodology and the NEBuilder Assembly Tool (http://nebuilder.neb.com/) have been used as suggested by manufacturer's instructions. The pPtPuc3 containing the kanamycin and bleomycin (Sh-Ble) expressing cassettes were used as templates, for the backbone vector, the vector PmH4pMRP3YFP for the coding sequence of the YFP, and the *P. tricornutum* cDNA for the coding sequence of PtOvoA without the stop codon. Total RNA was extracted from wild type *P. tricornutum* axenic cultures and cDNA retrotranscription was performed as in (Russo, M. T., Annunziata, R., Sanges, R., Ferrante, M.I., Falciatore, A., 2015, The upstream regulatory sequence of the light harvesting complex Lhcf2 gene of the marine diatom Phaeodactylum tricornutum enhances transcription in an orientation- and distance-independent fashion. Mar. Biol. 24, 69-79). Sequences of the primers used are presented in **Table 1.** The assembled construct was transformed in MAX Efficiency^{®} Stbl2^{™} Competent Cells (Invitrogen) using kanamycin as selective antibiotic. Resistant clones were analysed by restriction digestion with Clal and agarose gel electrophoresis **(****Fig. 2****).**

### P. tricornutum conjugation with Escherichia coli

Axenic CCMP632 strain of *P. tricornutum* Bohlin was obtained from the Provasoli-Guillard National Center for Culture of Marine Phytoplankton. Culture were grown in f/2 medium (Guillard R R L 1975 Culture of Phytoplankton for Feeding Marine Invertebrates. In: Smith, M.L. and Chanley, M.H., Eds., Culture of Marine Invertebrates Animals, Plenum Press, New York, 29-60. Doi: http://dx.doi.org/10.1007/978-1-4615-8714-9_3) at 18 °C under white fluorescent lights (70 µmol m-2 s-1.), 12 h:12 h dark-light cycle. The obtained plasmid pPtPuc3ovoYFP (cargo vector) was chemically transformed in DH10B cells containing the conjugation vector pTA-Mob (conjugation vector), bearing the gentamycin resistance (Strand T A, Lale R, Degnes K F, Lando M, Valla S. 2014 A New and Improved Host-Independent Plasmid System for RK2-Based Conjugal Transfer. PLoS ONE 9: e90372. Doi: https://doi.org/10.1371/journal.pone.0090372). *E. coli* DH10B, resistant to gentamycin and kanamycin, containing conjugation and cargo vectors were inoculated the day before the conjugation for overnight growth and used for the conjugation of *P. tricornutum* performed as in Karas, B., Diner, R., Lefebvre, S. et al. 2015 Designer diatom episomes delivered by bacterial conjugation. Na.t Commun 6, 6925.

### Preparation of the biolistic overexpressing construct

To obtain the pPtLhcf2povoYFP **(****Fig. 3****)** expression construct the Gibson Assembly methodology and the NEBuilder Assembly Tool have been used, following manufacturer's instructions. The following templates have been used: pPtLhcf2pLAHYFP for the backbone vector containing the Lhcf2 promoter and the YFP coding sequence with the primers GA_Lhcf2 finid and GA_Lhcf2_rv **(Table 1),** and the *P. tricornutum* cDNA for the coding sequence of PtOvoA without the stop codon with the primers: Ovo GA fwd and OvorvAt **(Table 1).**

### P. tricornutum biolistic transformation

The construct pPtLhcf2povoYFP has been introduced in *P. tricornutum* cells by means of microparticle bombardment using a Biolistic PDS-1000/He Particle Delivery System (Bio-Rad) as described in (Falciatore, A., Casotti, R., Leblanc, C., Abrescia, C., Bowler, C., 1999, Transformation of Nonselectable Reporter Genes in Marine Diatoms. Mar. Biotech. 1, 239-251). The construct pPtLhcf2povoYFP has been co-transformed with the pFCPBp-Sh ble vector (Falciatore, A., Casotti, R., Leblanc, C., Abrescia, C., Bowler, C. 1999, Transformation of Nonselectable Reporter Genes in Marine Diatoms. Mar Biotech 1, 239-251), which expresses the Sh Ble gene conferring resistance to the phleomycin antibiotic (Invitrogen).

### Selection of resistant clones

Resistant clones for bacterial conjugation were selected on plates prepared with f/2 medium diluted with 50% distilled water, 1% agarose, phleomycin 20 µg/ml.

Resistant clones for the biolistic transformation were selected on plates prepared with f/2 -Si medium diluted with 50% distilled water, 1% agar, and phleomycin (50 µg/ml) containing plates.

Colony PCR was performed as in (Daboussi, F., Leduc, S., Maréchal, A. et al. 2014. Genome engineering empowers the diatom Phaeodactylum tricornutum for biotechnology. Nat. Commun. 5,3831) on cell lysates with Lhcf2pfw2 and Ovorv1 primer pair **(Table 1)** to ascertain the presence of the episome (pPtPuc3ovoYFP) in the cells obtained by bacterial conjugation, or with Lhcf2pfini - Ovo1rv and Ovo1fini - GFP dw primer pairs (Table 1) to ascertain the presence of the transgene in the cells transformed with the biolistic method.

The PCR products were analysed by electrophoresis on agarose gel **(****Fig. 4****).**

### OvoA subcellular localization

OvoA protein sequence was scanned using the online tool for subcellular localization prediction WoLF PSORT. After conversion of protein amino acid sequences into numerical localization features, WoLF PSORT uses a simple k-nearest neighbour classifier for prediction and gives a summary line where the numbers indicate the number of nearest neighbours to the query which localize to each site (Horton, P., Park, K.J., Obayashi, T., Fujita, N., Harada, H., Adams-Collier, C.J., Nakai, K., 2007, WoLF PSORT: protein localization predictor. Nucleic Acids Res 35, W585-7). For microscopic analysis Confocal Laser Scanning TCS SP8 Leica was used. Chlorophyll autofluorescence and YFP fluorescence were excited at 510 nm and detected at 650-740 nm and 540-560 nm, respectively. MitoTracker Orange with excitation at 554 nm and emission at 576 nm (Molecular Probes) was used for mitochondrion staining.

### Cell pellets production

10 liters cultures of wild type (Pt1) and PtOvoA overexpressing (PtOvoA-oe) *P*. *tricornutum* cells from the late exponential (4-6x10E6) growth phase were harvested by centrifugation at 2500 x g for 15 minutes and frozen in liquid nitrogen.

### Chemical Identification of Ovothiol

100 mg of freeze dried cells were rehydrated with 300 µL water. 10 mg of the rehydrated cells were spiked with 10 µL of 1 mM N-acetyl cysteine. 90 µL extraction buffer (acetonitrile:0.75 M perchloric acid, 1:2) were added to the samples which were then vortexed (3 x 20 sec.). The samples were centrifuged for 5 min at 14000 rpm to remove cellular debris. The cleared lysate was neutralized by the addition of 15 µL of 2 M potassium carbonate. The mixture was centrifuged for 2 min at 1400 rpm to remove insoluble potassium perchlorate. The supernatant was basified with 10 µL lithium carbonate (50 mM, pH 10). The mixtures were incubated for 5 minutes with 3 µL 100 mM DTT and then incubated for 30 minutes with 25 µL of 20 mM 4-bromomethyl-7-methoxycoumarin (BMC) in DMSO. The mixture was then acidified by the addition of 10 µL 10% formic acid. Samples were vortexed to remove CO2 and then centrifuged for 10 minutes. Samples were analyzed by reverse phase HPLC using a Gemini-NX C18 (5 µm, 250 x 4.6 mm) column on a Shimadzu Prominence series apparatus. Thiol-BMC conjugates were detected with a DA detector at 330 nm. The HPLC method was as follows: solvent A is H2O/ACN/TFA 100:1:0.1, solvent B is ACN/TFA 100:0.085. The flow rate was 1 ml x min-1. Linear gradients were used for separation (0 min, 2% B; 1.5 min, 2% B; 22.5 min, 70% B; 24.5 min, 95% B; 29.5 min, 95% B; in 31.5 min, 2% B; 34.5 min, 2% B). Peak areas were compared to the one of N-acetyl cysteine used as internal standard to quantify the amount of ovothiol B. Glutathione concentrations were analyzed as a control. HRMS was obtained from UPLC HRMS (m calculated for [C19H22N3O5S]+ = 404.1275, m measured = 404.1258, Δ = -4.2 ppm). Ovothiol B contents were calculated as µg/mg dry weight cells.

### Example 1:

### Cellular Localization of OvoA Protein

Ten out of 11 resistant clones from bacterial conjugation revealed an expected PCR amplified product of 1205 bp corresponding to a fragment of the region encompassing the *P. tricornutum* Lhcf2 promoter and the PtOvoA coding sequence **(****Fig. 4****).** The construct pPtPuc3ovoYFP contains the YFP coding sequence in frame with PtOvoA generating transgenic lines expressing OvoA fused to a YFP tag at the C-terminal under the regulation of the *Lhcf*2p promoter **(****Fig. 1****),** allowing the examination of PtOvoA cellular localization through confocal laser scanning microscopy. The cells showed a signal in the mitochondrion **(****Fig. 5****),** confirming the predicted *in silico* localization of *P*. *tricornutum* OvoA **(Table 2).**

**Table 1 List of the primers**

| | | |
|---|---|---|
| SEQ ID NO 1 | pPtPuc3_rev | 5’- TCTAGAGGTCAAGTCCA GACTC-3 |
| SEQ ID NO 2 | Lhcf2p_fwd | 5’- CAGGAGTCTGGACTTG ACCTCTAGAGGTACCCC GCTTTGGTTTC-3' |
| SEQ ID NO 3 | Lhcf2p_rev | 5’- GTGGAGAGAGCATCAT CTTGACTTCTGGCAACC G-3’ |
| SEQ ID NO 4 | Ovo_fwd | 5’- GCCAGAAGTCAAGATG ATGCTCTCTCCACAAG- 3' |
| SEQ ID NO 5 | Ovo_rev | 5'- CCTTGCTCACCATTCCT GTCTTACGCCAACC-3' |
| SEQ ID NO 6 | YFP-Lhcf1t _ fwd | 5’- GCGTAAGACAGGAATG GTGAGCAAGGGCGAG- 3' |
| SEQ ID NO 7 | YFP- Lhcf1t _rev | 5’- CAAGCTTGCATGCCTGC AGGTCGACGCTGAAGA CGAGCTAGTGTTATTC- 3' |
| SEQ ID NO 8 | pPtPuc3_fwd | 5'- GTCGACCTGCAGGCAT GC- 3' |
| SEQ ID NO 9 | Lhcf2pfw2 | 5'- CGCCGTAAACAGCAAAT CCT-3' |
| SEQ ID NO 10 | Ovorv1 | 5’- AAGGAAGGAAAGTCGG CAGG-3’ |
| SEQ ID NO 11 | GA_Lhcf2_fwd | 5'- GAATTCATGGTGAGCAA GGGCG-3' |
| SEQ ID NO 12 | GA_Lhcf2_rv | 5’- GAATTCGATATCAAGCTT ATCGAT-3’ |
| SEQ ID NO 13 | Ovo GA fwd | 5'- AAGTCAAGTCGACGGTA TCGATAAGCTTGATATC GAATTCATGATGC TCTCTCCACAAGC-3' |
| SEQ ID NO 14 | OvorvAt | 5’- CCAAAGTCGAGGTAGTT GTTTCATCCTGTCTTAC GCCAAC-3’ |
| SEQ ID NO 15 | Lhcf2pfw | 5’- CGCCGTAAACAGCAAAT CCT-3’ |
| SEQ ID NO 16 | GFP dw | 5’- AACTCCAGCAGGACCAT GTG-3’ |

**Table 2. WoLF PSORT output indicating the predicted localization. The numbers indicate the number of nearest neighbours to the query which localize to each site, adjusted to account for the possibility of dual localization. mito, mitochondria; plas, plasma membrane; cysk, cytoskeletal; extr, extracellular; nucl, nuclear; pero, peroxisome; lyso, lysosome; chlo, chloroplast.**

| | |
|---|---|
| Animal | mito: 25, plas: 2.5, cysk_plas: 2, extr: 1, nucl: 1, pero: 1, lyso: 1 |
| Plant | mito: 13, chlo: 1 |
| Fungi | mito: 18, extr: 5, cyto: 2, pero: 2 |

### Example 2

### Thiol compounds analysis in P. tricomutum and identification of ovothiol B

The OvoA localization in *P. tricornutum* indicated that PtOvoA is translated in the diatom's cells and targeted to mitochondria **(****Fig. 5****).** To verify that PtOvoA cells produced ovothiol, we examined the thiol content of *P*. *tricornutum* cells cultured under physiological conditions and collected during their exponential phase. Extracted thiols were alkylated with the thiol-specific electrophilic dye 4-bromomethyl-7-methoxycoumarin (BMC) and analyzed by reversed phase HPLC. Glutathione was identified as the main thiol with a concentration of 1.5 µg/mg dry weight. Conversely, no traces of known alternative thiols such as ovothiol A or ergothioneine were detected. However, a signal was identified as the BCM-alkylated derivatives of ovothiol B by HR-ESI-MS (m/z_calc.= 404.1275, measured m/z = 404.1258, Δ < 5 ppm) and by co-elution with an authentic sample **(****Fig. 6****).** The level of ovothiol B was 0.5 µg/mg dry weight in PtWT. Transgenic lines expressing OvoA PtOvoA by bacterial conjugation, while exhibiting the same concentration of glutathione levels as in PtWT, produced at least a double amount of ovothiol B. PtOvoA obtained by the biolistic method produced a three to five-fold increase amount of ovothiol B compared to PtWT

### Example 3

### PtOvoA overexpression by means of the biolistic method

We analysed the cells of the resistant clones from biolistic method displaying a single band of expected size in the bottom panel of Figure 7 through confocal laser scanning microscopy and all of them confirmed PtOvoA cellular localization in the mitochondrion **(****Fig. 8****).**

### Sequence listing part of the description

SEQ ID NO 1 - pPtPuc3_rev primer
TCTAGAGGTCAAGTCCAGACTC

SEQ ID NO 2 - Lhcf2p_fwd primer
CAGGAGTCTGGACTTGACCTCTAGAGGTACCCCGCTTTGGTTTC

SEQ ID NO 3 - Lhcf2p_rev primer
GTGGAGAGAGCATCATCTTGACTTCTGGCAACCG

SEQ ID NO 4 - Ovo_fwd primer
GCCAGAAGTCAAGATGATGCTCTCTCCACAAG

SEQ ID NO 5 - Ovo_rev primer
CCTTGCTCACCATTCCTGTCTTACGCCAACC

SEQ ID NO 6 - YFP-Lhcf1t _fwd primer
GCGTAAGACAGGAATGGTGAGCAAGGGCGAG

SEQ ID NO 7 - YFP- Lhcf1t _rev primer
CAAGCTTGCATGCCTGCAGGTCGACGCTGAAGACGAGCTAGTGTTATTC

SEQ ID NO 8 - pPtPuc3_fwd primer
GTCGACCTGCAGGCATGC

SEQ ID NO 9 - Lhcf2pfw2 primer
CGCCGTAAACAGCAAATCCT

SEQ ID NO 10 - Ovorv1 primer

AAGGAAGGAAAGTCGGCAGG

SEQ ID NO 11 - GA_Lhcf2_fwd primer

GAATTCATGGTGAGCAAGGGCG

SEQ ID NO 12 - GA_Lhcf2_rv primer

GAATTCGATATCAAGCTTATCGAT

SEQ ID NO 13 - Ovo GA fwd primer

AAGTCAAGTCGACGGTATCGATAAGCTTGATATCGAATTCATGATGC
TCTCTCCACAAGC

SEQ ID NO 14 – OvorvAt primer

CCAAAGTCGAGGTAGTTGTTTCATCCTGTCTTACGCCAAC

SEQ ID NO 15 - Lhcf2pfw primer

CGCCGTAAACAGCAAATCCT

SEQ ID NO 16 - GFP dw primer

AACTCCAGCAGGACCATGTG

SEQ ID NO 17 – PtovoA peptidic sequence

MMLSPQAARMGTVCTTLRKIVSTARQRSLSHSAFASVRSETAFGARAFSTRAKSTLA
ALEDSDDEDLTFRSQGHAAAAAALSKAGLNKSHDEAWMINVNRNDDNEWLNGPRSA
EWFTGIHPSQCPGADQAGTIRSLSLPNLSAVTREAAKEYFDNSWTLYETLFAGLKGEE
GFYRPPVHGLRHPQIFYYGHTACLYINKLRVSKVLPKPVNAYFESIFEVGVDEMLWDD
MNKNDMLWPTVSEVHEYRQQVYKTVVDAILNHPSLDQRNGPVKVDQDHPMWALFM
GFEHERIHMETSSVLFRETPYHLVQTPQHWPPIHPSAFNDASPTSNPIETLDYPANRM
IAVDNGTVDLGKPADFPSFGWDNEYGERNMDVPPFFASEHMITNGEYWQFVDNGGY
RNREYWCDDGWAWRSHRNLKWPFFWEPAGPAGSNKFSLRTIFKIVPMPWSWPVDV
NYYEAQAFCRWKTEKEGSPTSKPYRILTEAEHHIIRNHDHNLEAARRDVSADKVMVTS
GQAFPKGSAGSNLNLAFSSQNPVDFFEPSQTGHRDTTGSAWEWTEDHFNPLKGFEV

HHVYDDFSTPCFDGKHSIIVGGSFISTGDEASVFARFHFRPHFLQHSGFRLVASDHDA
PATHLFAGNFDGQVAARDAAVAQEESKPRQSSLGSGSGSGNVYETDDSLHMYLGLH
YPNSGEKEGVAPILPHDNSPNHGTGFPQRVAGLLSSLKPEFNNNRALDIGCAVGGAS
FELAKTFDHVDAFDFSGSFVSAAKRMQSAENIKFRVPVEAELYEDLQAIHEHGVTDSV
RSKVQFFTGDACRLIDMKEDGILGSYDGVVMSNLLCRLPDPMACLAGLPEIINPGGVV
VMVTPFSWLTEFTPRGKWLGGFYDPVTNEAIYSKDILRQIMASNGFEKIHEVQMPLVI
REHQRKYQYIVSEATGWRKTG

SEQ ID NO 18 – PtovoA nucleotidic sequence
atgatgctctctccacaagccgcccgtatgggtaccgtgtgcacgacactccgcaagatagtttcgacggcgcgtcagcg
gtctctctcgcattcggcgttcgcgtcggtccgtagcgagaccgcgtttggagcccgggcgttttcgacgcgtgcgaaatct
actctagcggcactcgaagactcggatgacgaggatctcacctttcgtagtcagggacacgccgcagccgccgcggctt
tgagcaaggcaggattgaacaagtctcacgatgaggcctggatgatcaacgtcaatcgcaacgacgacaacgaatgg
ctgaacgggccgcgcagtgccgaatggtttacgggcattcaccctagtcaatgtcctggagccgaccaagcgggcacc
atccgctcgctttcacttccgaatctgtcggccgttacccgtgaagctgcaaaagaatactttgacaactcatggacgctcta
cgagacattgtttgccggtctcaaaggagaagaaggattttatcgcccgccagtccacggtctccgccacccccagatatt
ctactacggacacactgcttgtctctacatcaacaagctccgcgtcagtaaagtcttacccaaacctgtgaacgcctatttc
gagtccatcttcgaagtcggtgtagacgaaatgctctgggatgacatgaacaagaatgatatgctttggcccacagtttcg
gaggtgcacgagtatcgacagcaagtatacaaaacggttgtggacgctattttgaatcatcctagtcttgatcaaaggaac
ggtccagtgaaagtcgatcaggatcatccaatgtgggcattgttcatgggcttcgaacacgagcggatccatatggaaac
gagttcagtcttgttccgtgagacgccgtaccatttggtccaaacaccccagcattggcctccgattcatccgtcggctttca
acgatgcctcgcccacaagcaatccgatagaaactttggattaccccgcgaaccgcatgattgccgtggacaatggaa
ccgtcgatctcggaaagcctgccgactttccttcctttggatgggacaatgagtacggtgaacgcaatatggatgtgcctcc
attcttcgctagtgaacacatgatcacaaatggagaatactggcagtttgtcgacaatggtggctatcgaaatcgagagta
ttggtgcgacgacggctgggcgtggcgcagtcatcgcaatcttaagtggccttttttttgggagcccgcaggacccgctgg
gtccaataagttttcgttgcgaaccattttcaagatcgttcccatgccgtggagttggcctgttgatgtaaattactacgaagc
gcaagccttctgtcgatggaagaccgagaaagaaggatctccgacttcaaaaccgtatagaattctcaccgaagcgga
gcatcacatcattcgaaaccacgatcacaacttggaggctgctcgtagagacgtttcggcggataaggtgatggtgactt
cagggcaagcgtttcccaaaggatcggctggatcaaatttgaacctggcattctctagccaaaaccccgtcgatttctttga
gccgtcccaaactggccaccgtgataccaccggaagtgcctgggaatggacggaagaccacttcaaccctttaaagg
gatttgaagtccaccacgtgtacgatgatttttccactccatgttttgatggcaagcactctatcattgtggggggatcttttata
agtactggcgacgaggcatcagtttttgcacgattccatttccgaccccatttcctacaacattctggtttccgtctggttgcatc
agatcacgatgctcctgctacgcacctttttgccggaaatttcgatggtcaagttgccgcacgcgatgccgcggtcgcgca
ggaagaatccaagccgagacagtcttcattaggaagcggcagtggcagcggcaatgtttacgagacggatgacagctt
gcatatgtatcttggccttcattaccctaattctggcgagaaggaaggcgttgccccgatccttcctcacgacaactctccaa

accatggaactggcttcccgcaacgagtggcaggtcttctgtcctcactgaaacccgagttcaataacaatcgcgcattg
gatattggctgtgctgttggaggggcgtcttttgaacttgctaagactttcgatcacgtggacgcctttgatttcagtggatctttc
gtgagcgctgccaagcgaatgcaatcggcagaaaatatcaagtttcgggttcccgtggaggctgaactatatgaagacc
ttcaggctatccacgaacatggtgtgacggattcggtgcgctccaaagtgcagttctttacaggggacgcctgccgactca
tcgatatgaaagaagacggaattcttggctcatatgatggcgtggtcatgtcgaatttactctgccgcctcccagatccaat
ggcatgcctcgccggacttccagagattataaatcccggtggagtggtcgtgatggtgacgccattttcgtggttgactgag
ttcaccccccggggcaaatggctaggaggattttacgaccccgtaacaaacgaagctatctattcgaaggacatcctgc
gccaaattatggcctcgaatgggttcgagaagattcatgaggttcagatgccgctcgtcattcgggagcaccaacgtaaa
taccagtatattgtcagcgaagctacaggttggcgtaagacaggatga

## Claims

1. A genetically modified microalgae, **characterized in that** said microalgae includes one or more copy of an overexpression cassette encoding for the 5-histidylcysteine sulfoxide synthase OvoA enzyme, wherein said 5-histidylcysteine sulfoxide synthase OvoA enzyme has SEQ ID NO:17 or isoforms or mutant sequences thereof with a sequence identity between 90-99% and the same enzymatic activity.

2. The genetically modified microalga according to claim 1, wherein said microalga is a diatom, preferably said diatom is of the species *Phaeodactylum tricornutum,* still more preferably said diatom is the strain Axenic CCMP632 of *P*. *tricornutum.*

3. The genetically modified microalga according to any one of claims from 1 to 2, wherein said overexpression cassette encoding for 5-histidylcysteine sulfoxide synthase OvoA enzyme comprises a nucletodic sequence having SEQ ID NO: 18..

4. The genetically modified microalga according to any one of claims from 1 to 3 obtainable by a method according to any one of the claims from 5 to 11.

5. A method for the preparation of a genetically modified microalga, comprising a step of transformation of said microalga with an exogenous sequence encoding for the 5-histidylcysteine sulfoxide synthase OvoA enzyme, wherein said sulfoxide synthase OvoA enzyme has SEQ ID NO:17 or isoforms or mutant sequences thereof with a sequence identity between 90-99% and the same enzymatic activity.

6. The method according to claim 5, comprising a step of transformation of said microalga with an expression vector comprising a nucleotide sequence encoding for the 5-histidylcysteine sulfoxide synthase OvoA enzyme.

7. Method according to claim 5 to 6 wherein said expression vector is introduced in said microalga by biolistic method or bacterial conjugation.

8. Method according to any one of claims 5 to 7, wherein said expression vector is an episome vector in said algal cell.

9. Method according to claims 5 to 8, wherein said expression vector is introduced in said microalga by bacterial conjugation.

10. Method according to claim 9, comprising a step wherein said expression vector is transformed in said bacteria cells, preferably *Escherichia coli* DH10B cells.

11. Method according to claims 5 to 8, wherein said expression vector is introduced in said microalga by biolistic method, preferably said biolistic method is performed using a Biolistic Particle Delivery System.

12. An expression vector suitable for the transformation of microalgae, comprising a nucleic acid sequence encoding for the 5-histidylcysteine sulfoxide synthase OvoA enzyme wherein said sulfoxide synthase OvoA enzyme has SEQ ID NO: 18.

13. Expression vector according to claim 12, wherein said expression vector is selected from a plasmid, a yeast vector, a mammalian vector, a viral vector, a single-stranded phage, a double-stranded phage, artificial chromosome.

14. Expression vector according to claims 12 and 13, wherein said expression vector is an episomal plasmid.

15. A method for the production of ovothiol(s) comprising the steps of:
i) culturing a genetically modified microalgae according to anyone of claims 1 to 3,
ii) harvesting said genetically modified microalgae and
iii) identification and/or quantification of the ovothiol(s) from said genetically modified microalgae obtained on step ii).
